# FASCICULE DE BREVET EUROPEEN

(11) **EP 2 254 551 B1**
(45) Date de publication et mention de la délivrance du brevet: **06.11.2013**
(21) Numéro de dépôt: 09716007.1
(22) Date de dépôt: 26.02.2009
(51) Int. Cl.: A61K 9/06, A61K 31/70, A61K 47/04, A61K 31/717, A61K 31/047, A61K 8/25, A61K 8/34, A61K 8/60, A61K 8/73, A61P 17/02, A61Q 19/00, A61K 47/02, A61K 47/26, A61K 47/38, A61K 9/00

(54) **Gel de sucralfate sterilisé**
Sterilisiertes Sucralfatgel
Sterilized sucralfate gel

(30) Priorité: 26.02.2008 FR 0851206
(43) Date de publication de la demande: 01.12.2010
(73) Titulaire: Pierre Fabre Dermo-Cosmétique, 92100 Boulogne-Billancourt (FR)
(72) Inventeur: CHESNOY, Sophie, F-59130 Lambersart (FR); DELAUNOIS, Marlène, F-31290 Cessales (FR); ROUGE, Sandrine, F-31860 Pins-Justaret (FR)
(74) Mandataire: Regimbeau
(86) Numéro de dépôt international: PCT/EP2009/052251
(87) Numéro de publication internationale: WO 2009/106555

(56) Documents cités:
- EP-A- 0 136 100
- EP-A- 0 402 933
- EP-A- 0 471 967
- WO-A-96/05843
- WO-A-02/089818
- WO-A1-01/72310
- DATABASE CA [Online] CHEMICAL ABSTRACTS SERVICE, COLUMBUS, OHIO, US; 9 novembre 2006 (2006-11-09), XP002501270 extrait de STN Database accession no. 145: 425833 & BG 108 153 A (BALKANFARMA TROYAN) 30 avril 2005 (2005-04-30)
- J. SWARBRICK ED.: "Encyclopedia of pharmaceutical technology, vol.4" 2007, INFORMA HEALTHCARE , US 327830 , XP002501269 page 2286 - page 2297
- K. KING: "Changes in the functional properties and molecular weight of sodium alginate following gamma irradiation" FOOD HYDROCOLLOIDS, vol. 8, no. 2, 1994, pages 83-96, XP008097289
- P. COLOMBO: "Sucralfate gel: the innovative capability of pharmaceutics." ACTA TOXICOLOGICA ET THERAPEUTICA, vol. XVI, no. 1/2, 1995, XP008097273 IT
- LAMBERS H ET AL: "Natural skin surface pH is on average below 5, which is beneficial for its resident flora", INTERNATIONAL JOURNAL OF COSMETIC SCIENCE, vol. 28, no. 5, October 2006 (2006-10), pages 359-370, ISSN: 0142-5463

## Description

La présente invention concerne un gel de sucralfate stérilisé par autoclave, ainsi qu'une composition pharmaceutique ou cosmétologique comprenant ce gel, tel qu'un pansement liquide stérile, et son utilisation comme cicatrisant de la peau.
De nombreux pansements liquides existent actuellement sur le marché. Ils sont généralement utilisés pour permettre la cicatrisation de petites plaies du visage ou du corps.

Dans le domaine de la cicatrisation de plaies plus importantes, particulièrement des escarres et ulcères, divers pansements liquides existent également. Parmi ceux-ci existent les pansements à base d'hydrogels, dont l'influence sur l'épithélisation des blessures est connue (Acta Derm. Venereol. 1998, 78, p.119-222).

Aux substances gélifiantes utilisées, telles que la carboxyméthylcellulose, les gommes xanthane ou guar ou les alginates, ainsi qu'à l'eau, qui sont les constituants essentiels de ces gels, peuvent être ajoutées des substances humectantes, telles que la glycérine, le propylène glycol ou le sorbitol, ainsi que des composés à action cicatrisante comme par exemple le sucralfate.

Cependant, ces pansements sont rarement stériles. La stérilisation évite cependant le recours à des conservateurs souvent irritants comme l'acide sorbique. Or, les escarres et les ulcères sont la conséquence de pathologies complexes chez des patients fragilisés et donc sensibles à tout risque d'allergie et d'infection. Le milieu hospitalier préfère donc l'utilisation de pansements liquides stériles.

Cependant, la stérilisation de tels gels n'est pas évidente. Le procédé choisi doit l'être de manière à ce qu'aucun composé actif ne soit dégradé pendant ou après l'opération de stérilisation, et à ce que les différents composés ne réagissent pas entre eux.

Le sucralfate est un sel d'aluminium de l'octasulfate de sucrose. Il est connu pour améliorer la cicatrisation et est couramment utilisé dans le traitement des ulcères gastrique et duodenaux et même des brûlures (Burns 2001,27,p.465-469)

Plusieurs brevets décrivent l'utilisation par voie topique du sucralfate sous forme de gel dans la cicatrisation.

FR 2646604 décrit une composition de sucralfate sous forme de gel. La solution de sucralfate est acidifiée à pH proche de 4,5 pour obtenir une composition stable dotée des propriétés anti-inflammatoires et cicatrisantes. Cependant, ce brevet ne décrit pas de gel stérile de sucralfate.

EP 402933 décrit un gel humide, solide ou liquide, de sucralfate obtenu à partir de sucralfate particulaire, le diamètre moyen des particules devant être inférieur à 6µm et leur surface spécifique supérieure à 200m²/g, et pouvant être associé à un principe actif du type amikacine. D'après les exemples, ce gel peut être stérilisé par rayon gamma. Cependant, les indications données dans ce brevet n'ont pas permis de reproduire le gel de sucralfate humide et seule une composition pâteuse a pu être obtenue et non un gel. De ce fait, aucun gel stérile n'a pu être reproduit par le procédé décrit dans ce brevet.

Les inventeurs ont découvert que l'autoclave est le seul moyen de stérilisation connu compatible avec le sucralfate.

En effet, après différents tests, il est apparu que le sucralfate, aussi bien sous forme de poudre que de gel, n'est pas stable lors d'une stérilisation par rayonnement gamma puisque la composition jaunit. De même, lors d'une stérilisation par l'oxyde d'éthylène, une adsorption importante de ce dernier sur le sucralfate rend nécessaire une phase de désorption longue et coûteuse afin d'éviter la présence de résidus toxiques impropre à l'utilisation souhaitée (pharmaceutique ou cosmétologique) du sucralfate.

Par ailleurs, les inventeurs ont constaté que le pH du gel à stériliser était un facteur important puisque des gels ayant un pH inférieur à 6 ne présentent pas une bonne stabilité après stérilisation (jusqu'à 75% de dégradation du sucralfate utilisé) alors que les gels de pH supérieur ou égal à 6 restent stables après stérilisation (moins de 10% de dégradation du sucralfate utilisé).

La présente invention a donc pour objet un gel stérilisé par autoclave comprenant du sucralfate et au moins 15% en poids d'un agent humectant choisi parmi le sorbitol, le glycérol et le propylène glycol, par rapport au poids total du gel, et de pH supérieur ou égal à 6.

En effet, il a pu être constaté qu'avec de telles conditions de pH et de dosage d'agent humectant, un gel stable de sucralfate était obtenu, pouvant supporter la stérilisation thermique par autoclave. Ainsi, un tel gel de sucralfate reste stable pendant et après stérilisation.

Cela réside probablement dans le fait que, en réglant ainsi le pH et la quantité d'agent humectant, la solubilisation du sucralfate est limitée ce qui doit le protéger de la dégradation à fortes chaleurs.

Avantageusement, le pH du gel de l'invention sera compris entre 6 et 7, et de préférence entre 6 et 6,8.

Dans un mode de réalisation particulier, le gel de l'invention comprend de 5 à 15%, et de préférence 10% en poids de sucralfate, par rapport au poids total du gel, afin de permettre une efficacité meilleure sur la cicatrisation.

De préférence, on choisira comme agent humectant le sorbitol qui est moins irritant et sensibilisant.

Avantageusement, le gel de l'invention contiendra de 15 à 25%, de préférence, de 15 à 20%, et encore de préférence de 15 à 18%, d'agent humectant.

Par ailleurs, le glycérol sera déconseillé pour les patients diabétiques.

Avantageusement, le gel de l'invention sera un hydrogel et donc pourra comprendre au moins 60%, et de préférence, au moins 69% en poids d'eau, par rapport au poids total de gel.

Afin d'obtenir une composition sous forme de gel, un agent gélifiant pourra également être ajouté au gel de l'invention, et notamment en quantité de 0,5% à 6% en poids, par rapport au poids total du gel.

L'agent gélifiant pourra notamment être choisi parmi l'hydroxyéthyl cellulose, l'hydroxypropyl cellulose, l'hydroxypropyl méthyl cellulose et la silice, et de préférence sera l'hydroxyéthyl cellulose ou la silice.

La silice utilisée pourra être en particulier de la silice colloïdale telle que l'Aérosil® V200 commercialisée par Degussa.

Avantageusement, l'agent gélifiant sera l'hydroxyéthyl cellulose.

Afin d'obtenir un gel avec le pH désiré, le gel de l'invention pourra comprendre également un acide ou une base pharmaceutiquement acceptable.

Par « acide pharmaceutiquement acceptable », on entend au sens de la présente invention tout acide non toxique, y compris les acides organiques et inorganiques. De tels acides incluent l'acide chlorhydrique, l'acide bromhydrique, l'acide sulfurique, l'acide nitrique, l'acide phosphorique, l'acide acétique, l'acide benzènesulfonique, l'acide benzoïque, l'acide camphresulfonique, l'acide citrique, l'acide éthane-sulfonique, l'acide fumarique, l'acide glucoheptonique, l'acide gluconique, l'acide glutamique, l'acide glycolique, l'acide hydroxynaphtoïque, l'acide 2-hydroxyéthanesulfonique, l'acide lactique, l'acide maléique, l'acide malique, l'acide mandélique, l'acide méthanesulfonique, l'acide muconique, l'acide 2-naphtalènesulfonique, l'acide propionique, l'acide salicylique, l'acide succinique, l'acide dibenzol-L-tartrique, l'acide tartrique, l'acide p-toluènesulfonique, l'acide triméthylacétite et l'acide trifluoroacétique.

Par « base pharmaceutiquement acceptable », on entend au sens de la présente invention toute base non toxique, y compris les acides organiques et inorganiques.

A titre de base inorganique, on peut citer des bases formant, par exemple, des sels d'ammonium ou des sels de métaux alcalins ou alcalino-terreux, tels que le lithium, le sodium, le potassium, le magnésium ou encore le calcium, comme notamment l'hydroxyde d'aluminium, l'hydroxyde de calcium, l'hydroxyde de potassium, le carbonate de sodium ou l'hydroxyde de sodium.

A titre de base organique, on peut citer, par exemple, la diéthanolamine, l'éthanolamine, N-méthylglucamine, la triéthanolamine, la triéthylamine ou encore la trométhamine.

Selon une caractéristique particulière de l'invention, le gel stérilisé de sucralfate ne contient pas de conservateur, et en particulier des conservateurs pouvant être absorbés par passage systémique.

Par « conservateur », on entend notamment le parahydroxybenzoate de méthyle, le parahydroxybenzoate de propyle, le parahydroxybenzoate de butyle, le phénoxyéthanol, la chlorphénésine ou encore l'acide sorbique.

Selon un mode de réalisation particulier, la stérilisation par autoclave du gel de l'invention pourra être réalisée à une température de 121 °C pendant une durée de 15 minutes, dans des conditions bien connues de l'homme du métier. Une telle stérilisation est ainsi réalisée sous vapeur d'eau saturante.

La présente invention a également pour objet une composition pharmaceutique ou cosmétologique comprenant un gel stérilisé de sucralfate tel que défini ci-dessus pour une application topique.

En particulier, une telle composition pourra être un pansement liquide stérile.

La présente invention a également pour objet l'utilisation d'un gel stérilisé de sucralfate tel que défini ci-dessus pour la préparation d'une composition pharmaceutique destinée à un traitement cicatrisant de la peau, et notamment d'escarres et/ou d'ulcères.

### EXEMPLES

### 1. Influence de la dose de sucralfate

Deux hydrogels comprenant respectivement 5 et 10% de sucralfate en poids et de pH égal à 4 ont été préparés avec la composition suivante :

| Composition | Hydrogel 1* | Hydrogel 2* |
|---|---|---|
| Sucralfate | 5 g | 10 g |
| Aerosil® V200 | 10,5 g | 9 g |
| Sorbitol | 16,45 g | 10,5 g |
| Eau totale | Qsp 100 g | Qsp 100 g |

| | | |
|---|---|---|
| (Qsp = quantité suffisante pour) * exemple comparatif | | |

Ces deux hydrogels ont alors été stérilisés en autoclave à 121 °C pendant 15 min et les résultats suivants ont été obtenus :

| Tests analytiques | Normes | Hydrogel 1 | Hydrogel 2 |
|---|---|---|---|
| pH avant autoclave | 4,0 ± 0,5 | 3,95 | 4,1 |
| pH après autoclave | 4,0 ± 0,5 | 3,62 | 3,75 |
| Dosage sucralfate avant stérilisation | 1,4 à 1,5% ou 9,0 à 11,0% | 1,6%* | |
| | | | 10,0% |
| Dosage sucralfate après | 1,4 à 1,5% ou | 0,4%* | |

| | | | |
|---|---|---|---|
| stérilisation | 9,0 à 11,0% | | 6,40% |

*Dans ce cas, le dosage du sucrafate correspond au dosage de l'octasulfate de sucrose qui compose le sucralfate à hauteur de 30 à 38% généralement.

On constate donc que la dégradation du sucralfate pour des compositions formulées à pH = 4 s'élève à 75% pour la première formulation comprenant 5% de sucralfate alors qu'elle n'est que de 36% pour la seconde formulation comprenant 10% de sucralfate.

Ainsi, lorsque la teneur en sucralfate est plus faible dans la composition, ce qui augmente sa solubilisation en milieu aqueux, sa dégradation est également plus élevée.

### 2 . Influence du pH

Deux nouveaux hydrogels comprenant 10% de sucralfate ont été préparés à un pH plus élevé égal à 6 avec la composition suivante :

| Composition | Hydrogel 3 | Hydrogel 4* |
|---|---|---|
| Sucralfate | 10 g | 10 g |
| Hydroxyéthylcellulose | 4 g | 4 g |
| Hydroxyde de sodium | 0,276 g | 0,276 g |
| Sorbitol | 16,45 g | 10,5 g |
| Eau totale | Qsp 100 g | Qsp 100 g |

| | | |
|---|---|---|
| * exemple comparatif | | |

Ces deux hydrogels ont alors été stérilisés en autoclave à 121 °C pendant 15 min et les résultats suivants ont été obtenus :

| Tests analytiques | Normes | Hydrogel 3 | Hydrogel 4 |
|---|---|---|---|
| pH avant autoclave | 6,0 ± 0,5 | 6,08 | 6,2 |
| pH après autoclave | 6,0 ± 0,5 | 5,42 | 5,5 |
| Dosage sucralfate avant stérilisation | 9,0 à 11,0% | 9,44% | 9,52% |
| Dosage sucralfate après stérilisation | 9,0 à 11,0% | 9,25% | 8,68% |

On constate donc que la stabilité du sucralfate pendant la stérilisation est améliorée avec des formulations à pH plus élevé puisque la dégradation du sucralfate dans les hydrogels 3 et 4 est inférieure à 10% dans les deux cas.

De plus, on constate également que la stabilité est légèrement meilleure lorsque la teneur en sorbitol, agent humectant, dans la composition est plus importante.

### 3. Influence de la dose d'agent humectant

Des études de stabilité accélérée ont été menées pendant 4 mois à 40 °C sur les hydrogels 3 et 4 précédents et ont conduit aux résultats suivants :

| Tests analytiques | Normes | Hydrogel 3 | Hydrogel 4 |
|---|---|---|---|
| Dosage après 4 mois à 40 °C | 9,0 à 11,0% | 9,21% | 7,35% |

On constate donc que l'hydrogel ayant une teneur en sorbitol, agent humectant, de plus de 15% reste stable dans le temps contrairement à l'hydrogel ayant une teneur en sorbitol de 10%.

## Revendications

1. Gel stérilisé par autoclave comprenant du sucralfate et au moins 15% en poids d'un agent humectant choisi parmi le sorbitol, le glycérol et le propylène glycol, par rapport au poids total de gel, et de pH supérieur ou égal à 6.

2. Gel selon la revendication 1, **caractérisé en ce qu'**il comprend de 5 à 15%, et de préférence 10% en poids de sucralfate, par rapport au poids total de gel.

3. Gel selon l'une des revendications 1 et 2, **caractérisé en ce que** l'agent humectant est le sorbitol.

4. Gel selon l'une des revendications 1 à 3, **caractérisé en ce qu'**il comprend au moins 60%, et de préférence au moins 69% en poids d'eau, par rapport au poids total de gel.

5. Gel selon l'une des revendications 1 à 4, **caractérisé en ce qu'**il comprend également un agent gélifiant.

6. Gel selon la revendication 5, **caractérisé en ce qu'**il comprend de 0,5% à 6% en poids d'agent gélifiant, par rapport au poids total de gel.

7. Gel selon l'une des revendications 5 et 6, **caractérisé en ce que** l'agent gélifiant est choisi parmi l'hydroxyéthyl cellulose, l'hydroxypropyl cellulose, l'hydroxypropyl méthyl cellulose et la silice, et de préférence est l'hydroxyéthyl cellulose ou la silice.

8. Gel selon l'une des revendications 1 à 7, **caractérisé en ce qu'**il comprend un acide ou une base pharmaceutiquement acceptable.

9. Gel selon l'une des revendications 1 à 8, **caractérisé en ce qu'**il ne contient pas de conservateur.

10. Gel selon l'une des revendications 1 à 9, **caractérisé en ce que** la stérilisation par autoclave est réalisée à une température de 121°C pendant une durée de 15 minutes.

11. Composition pharmaceutique ou cosmétologique comprenant un gel selon l'une des revendications 1 à 10 pour une application topique.

12. Composition selon la revendication 11 en tant que pansement liquide stérile.

13. Utilisation d'un gel selon l'une des revendications 1 à 10 pour la préparation d'une composition pharmaceutique destinée à un traitement cicatrisant de la peau, et notamment d'escarres et/ou d'ulcères.

## Patentansprüche

1. Gel, sterilisiert mittels Autoklav, umfassend Sucralfat und mindestens 15 Gew.% eines Feuchthaltemittels, das ausgewählt ist aus Sorbit, Glycerin und Propylenglykol, bezogen auf das Gesamtgewicht des Gels, und einem pH oberhalb oder gleich 6.

2. Gel nach Anspruch 1, **dadurch gekennzeichnet, dass** es 5 bis 15 Gew.%, bevorzugt 10 Gew.% Sucralfat umfasst, bezogen auf das Gesamtgewicht des Gels.

3. Gel nach Anspruch 1 oder 2, **dadurch gekennzeichnet, dass** das Feuchthaltemittel Sorbit ist.

4. Gel nach einem der Ansprüche 1 bis 3, **dadurch gekennzeichnet, dass** es mindestens 60 Gew.%, bevorzugt mindestens 69 Gew.% Wasser umfasst, bezogen auf das Gesamtgewicht des Gels.

5. Gel nach einem der Ansprüche 1 bis 4, **dadurch gekennzeichnet, dass** es ferner ein Geliermittel umfasst.

6. Gel nach Anspruch 5, **dadurch gekennzeichnet, dass** es 0,5 bis 6 Gew.% Geliermittel umfasst, bezogen auf das Gesamtgewicht des Gels.

7. Gel nach Anspruch 5 oder 6, **dadurch gekennzeichnet, dass** das Geliermittel ausgewählt ist aus Hydroxyethylzellulose, Hydroxypropylzellulose, Hydroxypropylmethylzellulose und Siliziumdioxid, bevorzugt Hydroxyethylzellulose oder Siliziumdioxid.

8. Gel nach einem der Ansprüche 1 bis 7, **dadurch gekennzeichnet, dass** es eine pharmazeutisch akzeptable Säure oder Base umfasst.

9. Gel nach einem der Ansprüche 1 bis 8, **dadurch gekennzeichnet, dass** es kein Konservierungsmittel umfasst.

10. Gel nach einem der Ansprüche 1 bis 9, **dadurch gekennzeichnet, dass** die Sterilisation mittels Autoklav bei einer Temperatur von 121 °C während einer Dauer von 15 Minuten durchgeführt wird.

11. Pharmazeutische oder kosmetologische Zusammensetzung, umfassend ein Gel nach einem der Ansprüche 1 bis 10 für eine topische Anwendung.

12. Zusammensetzung nach Anspruch 11 als steriler Flüssigverband.

13. Verwendung eines Gels nach einem der Ansprüche 1 bis 10 für die Herstellung einer pharmazeutischen Zusammensetzung, die für eine vernarbende Behandlung bestimmt ist, und insbesondere von wunden Stellen und/oder Geschwüren.

## Claims

1. An autoclave-sterilized gel comprising sucralfate and at least 15% by weight of a humectant selected from sorbitol, glycerol and propylene glycol, relative to the total weight of the gel, and having a pH greater than or equal to 6.

2. The gel according to claim 1, **characterized in that** it comprises from 5% to 15%, and preferably 10% by weight of sucralfate, relative to the total weight of the gel.

3. The gel according to one of claims 1 and 2, **characterized in that** the humectant is sorbitol.

4. The gel according to one of claims 1 to 3, **characterized in that** it comprises at least 60%, and preferably at least 69% by weight of water, relative to the total weight of the gel.

5. The gel according to one of claims 1 to 4, **characterized in that** it further comprises a gelling agent.

6. The gel according to claim 5, **characterized in that** it comprises from 0.5% to 6% by weight of the gelling agent, relative to the total weight of the gel.

7. The gel according to one of claims 5 and 6, **characterized in that** the gelling agent is selected from hydroxyethyl cellulose, hydroxypropyl cellulose, hydroxypropyl methylcellulose and silica, and preferably is hydroxyethyl cellulose or silica.

8. The gel according to one of claims 1 to 7, **characterized in that** it comprises a pharmaceutically acceptable acid or base.

9. The gel according to one of claims 1 to 8, **characterized in that** it contains no preservative.

10. The gel according to one of claims 1 to 9, **characterized in that** the autoclave sterilization is carried out at a temperature of 121°C for a period of 15 minutes.

11. A pharmaceutical or cosmetic composition comprising a gel according to one of claims 1 to 10 for topical application.

12. The composition according to claim 11 as a sterile liquid bandage.

13. Use of a gel according to one of claims 1 to 10 for the preparation of a pharmaceutical composition intended for a healing treatment of the skin, and in particular of eschars and/or ulcers.
